(19) Europäisches Patentamt
European Patent Office
Office européen des brevets

(11) Veröffentlichungsnummer: **0 079 913**
**B1**

(12) # EUROPÄISCHE PATENTSCHRIFT

(45) Veröffentlichungstag der Patentschrift: **26.02.86**

(51) Int. Cl.⁴: **A 61 K 7/06**, A 61 K 7/48

(21) Anmeldenummer: **82901593.2**

(22) Anmeldetag: **21.05.82**

(86) Internationale Anmeldenummer: **PCT/EP 82/00109**

(87) Internationale Veröffentlichungsnummer: **WO 82/04189 (09.12.82 Gazette 82/29)**

(54) **ARENCARBONSÄURE-DERIVATE ALS ANTISEBORRHOISCHE ZUSÄTZE FÜR KOSMETISCHE MITTEL.**

(30) Priorität: **27.05.81 DE 3121064**

(43) Veröffentlichungstag der Anmeldung: **01.06.83 Patentblatt 83/22**

(45) Bekanntmachung des Hinweises auf die Patenterteilung: **26.02.86 Patentblatt 86/9**

(84) Benannte Vertragsstaaten: **AT BE CH DE FR GB LI LU NL SE**

(56) Entgegenhaltungen:
**BE - A - 524 131**
**BE - A - 802 042**
**CH - A - 561 544**
**DE - A - 2 617 817**
**FR - A - 893 112**
**NL - B - 57 704**
**US - A - 3 076 017**
**US - A - 3 317 382**

**Organische-Chemische Arzneimittel und ihre Synonyma, Band I (Auflage 5 (1978)), Seiten 137,184,185,235,301,358,437,438**

(73) Patentinhaber: **Henkel Kommanditgesellschaft auf Aktien, Postfach 1100 Henkelstrasse 67, D-4000 Düsseldorf-Holthausen (DE)**

(72) Erfinder: **MÖLLER, Hinrich, Schumannstrasse 11, D-4019 Monheim (DE)**
Erfinder: **WALLAT, Siegfried, Hasenstrasse 7, D-4019 Monheim (DE)**
Erfinder: **BARTNIK, Friedhelm, Melanchthonstrasse 11, D-4000 Düsseldorf (DE)**

EP 0 079 913 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Gegenstand der Erfindung sind topische, kosmetische Zubereitungen zur Verbesserung des fettigen und unästhetischen Aussehens der Haare und der Haut, insbesondere zur Behandlung von stark fettendem Haar.

Die übermäßig starke Absonderung der Talgdrüsen der Kopfhaut verleiht dem Haar ein fettiges Aussehen, das in allgemeinen als wenig ästhetisch angesehen wird. Es ist daher vielfach versucht worden, durch geeignete Mittel die Sekretion der Talgdrüsen zu normalisieren, um dem Haar wieder sein gesundes Aussehen zu geben. Zur Therapie der Seborrhoe wurden in der DE-OS 16 67 902 peroral verabreichbare Zubereitungen, welche Cysteamin-Derivate enthalten, vorgeschlagen. Es wurde zur Bekämpfung der Seborrhoe des behaarten Kopfes Shampoos mit Schwefel, Quecksilber oder Teerzusatz verwendet. Dabei hat sich gezeigt, daß diese bekannten Mittel bei längerer Anwendung häufig zu Nebenwirkungen führen, ohne daß wirklich befriedigende Ergebnisse im Hinblick auf Wirksamkeit oder anwendungstechnische Eigenschaften erzielt werden konnten. In der DE-OS 19 06 665 wurde schließlich zur Behandlung der durch Seborrhoe verursachten Schuppenbildung N,N-Diethyl-m-Toluamid als Wirkstoff vorgeschlagen.

In der US-PS 3 755 604 werden Phenylpentadien-Säuren als Mittel zur Verhinderung der Sebumproduktion vorgeschlagen. Es hat sich jedoch gezeigt daß weder N N-Diethyl-m-Toluamid noch Phenylpentadiensäuren zufriedenstellende antiseborrhoische Wirkungen zeigen.

Es wurde nun gefunden, daß topische, kosmetische Zubereitungen mit einem Gehalt an Verbindungen der allgemeinen Formel:

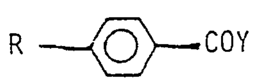 (I)

worin bedeuten

$R = C_{10}-C_{14}$-Alkoxy

$Y = C_1-C_{12}$-Alkoxy oder Aralkoxy

eine besonders bemerkenswerte Wirksamkeit bei der Behandlung seborrhoischer Haut und stark fettendem Haar aufweisen. Die Verbindungen der Formel (1) sind literaturbekannt bzw. lassen sich nach an sich bekannten Verfahren der organischen Chemie herstellen, z. B. durch Alkylierung von phenolische Hydroxylgruppen enthaltenden Arencarbonsäureestern mit geeigneten Alkylierungsmitteln.

Den erfindungsgemäß einzusetzenden Verbindungen zugrunde liegende Arencarbonsäuren sind zum Beispiel: 4-Decyloxy- oder 4-Tetradecyloxybenzoesäure. Geeignete Alkoholkomponenten für die Herstellung der erfindungsgemäß einzusetzenden Arencarbonsäureester sind zum Beispiel:

Methanol, Ethanol, Propanol, Isopropanol, n-Butanol, iso-Butanol,sek. Butanol, tert. Butanol, Pentanol, Hexanol, Octanol, 2-Ethylhexariol, Decanol, Benzylalkohol.

Die erfindungsgemäßen kosmetischen Mittel stellen Lösungen der einzusetzenden wirksamen Verbindungen der Formel (1) in Wasser, in Alkohol, in wäßrig-alkoholischen Michungen, in Öl, Suspensionen, Gelen, Emulsionen, Salben, Pasten oder Aerosoler dar. Die antiseborrhoischen Arencarbonsäureester können in fast alle zur Behandlung von Haut und Haaren üblichen kosmetischen Mitteln eingearbeitet werden, wie zum Beispiel in Haarwässer, Haarshampoos, Haarkuren, Haarspülungen, oder auch in Hautlotionen und Schüttelmixturen. Neben den Verbindungen der Formel (1) enthalten die erfindungsgemäßen Zubereitungen bekannte Träger und Hilfsstoffe wie Wasser, organische Lösungsmittel, oberflächenaktive Verbindungen, Öle und Fette, Wachse, Parfümöle, Farbstoffe, Konservierungsmittel und dergleichen. Eine vorteilhafte Anwendungsform zur Behandlung von stark fettendem Haar ist das Shampoo. Derartige Shampoos können neben dem sebosuppressiven Wirkstoff anionische, kationische, nichtionische oder amphotere Tenside, Farbstoffe, Duftstoffe, Verdickungsmittel oder Konditionierungsmittel enthalten. Die erfindungsgemäßen kosmetischen Zubereitungen enthalten die Arencarbonsäure-Derivate in einer Menge von 0,0l bis 20 Gewichtsprozent, vorzugsweise 1 bis 10 Gewichtsprozent bezogen auf das Gewicht der gesamten Zubereitung. Die erfindungsgemäßen Mittel können täglich angewendet werden; es werden jedoch bereits bei einmaliger wöchentlicher Anwendung zufriedenstellende Ergebnisse erzielt. Die individuelle Dosis, die bei jeder Behandlung angewendet werden sollte, ist nicht kritisch. Nachteilige Nebeneffekte wurden nicht beobachtet. Das fettige Aussehen des Haares wird vermindert und das Nachfetten verzögert, wodurch die Möglichkeit einer normalen Haarpflege gegeben ist. Bei Einsatz der erfindungsgemäßen Mittel in Form von Hautcremes oder Milchpräparaten oder Schüttelmixturen gelingt es, durch regelmäßige Anwendung auf der Haut deren Aussehen dauerhaft zu verbessern.

Das nachfolgende Beispiel soll den Gegenstand der Erfindung näher erläutern.

**Beispiel**

Zur Erläuterung des Verfahrens wird zunächst die Herstellung eines nicht beanspruchten chlorhaltigen Arencarbonsäureesters beschrieben:

3,4-Dichlor-benzoesäure-ethylester Eine Mischung aus 42 g (o,2 Mol) 3,4-Dichlorbenzoylchlorid und 200 ml Ethanol wurde 2 Stunden zum Sieden erhitzt, eingedampft, der Rückstand in Ether aufgenommen, die Lösung mit$NaHCO_3$ -Lösung . und Wasser gewaschen, getrocknet und eingedampft. Nach dem Destillieren wurden 32g/73% der Theorie) 3,4-Dichlor-benzoesäure-ethylester vom Kp. 84 C/0,13 mbar und Fp: 33 bis 35° C erhalten.

In analoger Weise wurde die folgende erfindungsgemäß verwendbare Verbindung hergestellt: 4-Decyloxybenzoesäuremethylester Fp.: 45 - 46° C

Die antiseborrhoische Wirkung der in den erfindungsgemäßen kosmetischen Zubereitungen eingesetzten Verbindungen wurde durch nachfolgend beschriebene Tierversuche näher untersucht. Als Versuchstiere dienten männliche Wistar-Ratten von 220 - 230 g, Körpergewicht. Beurteilt wurde visuell der Bräunungsgrad auf dem Rücken der dort geschorenen Ratten.Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung beziehungsweise einem Lipidlösungsmittel und auch männliche Ratten, die systemisch mit Östrogen behandelt wurden, nur die normale helle, rosafarbene Haut nach dem Scheren aufweisen; parallel dazu sind aus den abgeschnittenen Haaren nur noch vergleichsweise sehr geringe lipidmengen zu extrahieren.

Zur Beurteilung der sebosuppressiven Wirksamkeit wurde die Prüfsubstanz 4-Decyloxybenzoesäure methylester in Form einer 1 % igen Lösung in Ethanol oder Ethanol/Aceton (1:1) jeweils 6 Ratten halbseitig auf das Rückenfell gepinselt. Die andere Seite wurde nur mit dem Lösungsmittel, ohne Wirksubstanz behandelt (Kontrollseite).

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Als 1. Kriterium wurde bewertet, ob die Mehrheit der Beurteiler richtig die behandelte Seite erkannt haben, wobei wie folgt differenziert wurde:

| Zeichen | Anteil Beurteiler, die eine Wirkung erkannten |
|---|---|
| ++ | 100 % |
| + | > 50 %, 100 % |
| 0 | ≤ 50 % |

Als 2. Kriterium wurde der Unterschied zwischen rechter und linker Seite gewertet, wobei pro Beurteiler und Tier jeweils 1 Punkt zu vergeben war, und zwar in der Weise, daß die dunklere Seite mit 1 und die hellere mit 0 und bei Gleichheit beide Seiten mit 0,5 benotet wurden.

Als 3. Kriterium wurden außerdem noch die Intensitätsunterschiede der Brauntöne nach folgender Skala bewertet:

3 Punkte stark braun
2 Punkte mittel braun
1 Punkt schwach braun
0 Punkte keine Braunfärbung

Signifikante Differenzen zwischen unbehandelten und behandelter Seite nach der zweiten Bewertungsmethode zeigen die lokale Wirksamkeit einer Substanz an. Nach der dritten Bewertungsmethode werden die Punktsummendifferenzen zwischen den unbehandelten Kontrolltieren und jeweils den behandelten und unbehandelten Seiten der Versuchstiergruppe gebildet, wobei wiederum signifikante Differenzen zwischen Kontrolltieren und der behandelten Seite der Versuchstiere die Wirkung einer Substanz deut lich machen.

Parallel dazu ist in der Regel auch ein deutlicher Unterschied zwichen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß. Zur Differerizierung der Effekte

gemäß Beurteilungsmethode 2 und 3 wurde das folgende Schema verwendet

| Zeichen | Punktdifferenz |
|---|---|
| ++ | sehr groß (> 99,9 % Wahrscheinlichkeit) |
| + | signifikant (≥ 95 % Wahrscheinlichkeit) |
| (+) | deutlich aber < 95 % Wahrscheinlichkeit |

Die nachfolgende Tabelle zeigt die Bewertungsergebnisse nach vorgenanntem Schema für die untersuchten Substanzen.

Bewertung der sebosuppressiven Effekte

| Substanz | Bewertungsmethode 1 | 2 | 3 |
|---|---|---|---|
| 4-Decyloxy benzoesäuremethyl ester | ++ | ++ | ++ |
| N,N-Diethyl-m-toluamid (DE-OS 19 06 665) | + | (+) | (+)-0 |
| 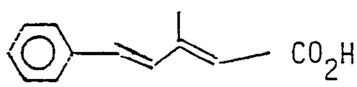 (US-PS 3 755 604) | 0 | 0 | + |

Nachfolgend wird ein Beispiel für eine erfindungsgemäße topische Zubereitung zur Behandlung von stark fettendem Haar und seborrhoischer Haut angegeben:

| Haarkur | Gewichts-Prozent |
|---|---|
| Tegin M(R) (Glycerinmono- und distearat) | o,7 |
| kationisches Tensid | 2,o |
| Cholesterin | o,2 |
| Sojalecithin | o,3 |
| Emulgade A(R) (Gemisch von Cetylstearylalkohol mit nichtionogenen Emulgatoren) | |
| Parfümöl | o,3 |
| 4-Decyloxybenzoesäuremethylester | 7,o |
| Wasser, vollentsalzt | 81,5 |

**Patentansprüche**

1. Topische, kosmetische Zubereitungen, enthaltend p-Alkoxybenzoesäurederivate der allgemeinen Formel

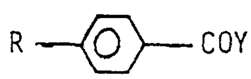
(I)

worin bedeuten

$R = C_{10}-C_{14}$-Alkoxy

$Y = C_1 - C_{12}$-Alkoxy oder Aralkoxy

als antiseborrhoische Wirkstoffe neben üblichen Träger- und Hilfsstoffen.

2. Topische, kosmetische Zubereitung nach Anspruch 1, dadurch gekennzeichnet, daß sie die Verbindungen der Formel (1) in einer Menge von 0,01 bis 20 Gewichtsprozent, vorzugsweise 1 bis 20 Gewichtsprozent, bezogen auf die gesamte Zubereitung enthält.

**Claims**

I. Topical cosmetic preparations containing p-alkoxybenzoic acid derivatives corresponding to the following general formula

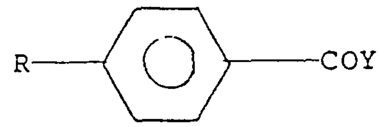

(I)

in which R = $C_{10}$-$C_{14}$ alkoxy Y = $C_1$-$C_{12}$ alkoxy or aralkoxy as antiseborrheic agents in conjunction with standard carriers and auxiliaries.

2. A topical cosmetic preparation as claimed in Claim 1, characterized in that it contains the compounds corresponding to formula (I) in a quantity of from 0.01 to 20% by weight and preferab.y in a quantity of from I to 20% by weight, based on the preparation as a whole.

**Revendications**

1. Préparations cosmétiques topiques, contenant des dérivés d'acides p-alcoxybenzoîques de formula générale:

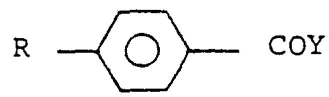

(I)

pour laquella
R = alcoxy en $C_{10}$-$C_{14}$
Y = alcoxy en $C_1$-$C_{12}$ ou aralcoxy en tant que matières actives anti-séborrhéiques à côté des matières de support et auxiliaires usuelles.

2. Préparation cosmétique topique selon la revendication 1, caractérisée en ca qu'elle contient les composés de formule (I) en une quantité de 0,01 à 20% an poids, de préférence de 1 à 20% en poids par rapport à la préparation totale.